# EUROPÄISCHE PATENTSCHRIFT

(11) **EP 2 217 304 B1**
(45) Veröffentlichungstag und Bekanntmachung des Hinweises auf die Patenterteilung: **28.03.2012**
(21) Anmeldenummer: 08846897.0
(22) Anmeldetag: 04.11.2008
(51) Int. Cl.: A61M 5/00

(54) **VORRICHTUNG UND VERFAHREN ZUR MONTAGE EINER PHARMAZEUTISCHEN APPLIKATIONSHILFE**
DEVICE AND METHOD FOR MOUNTING A PHARMACEUTICAL APPLICATION AID
DISPOSITIF ET PROCÉDÉ POUR LE MONTAGE D'UN SYSTÈME D'ADMINISTRATION DE MÉDICAMENT

(30) Priorität: 07.11.2007 DE 102007054868
(43) Veröffentlichungstag der Anmeldung: 18.08.2010
(73) Patentinhaber: Arzneimittel GmbH Apotheker Vetter & Co. Ravensburg, 88212 Ravensburg (DE)
(72) Erfinder: KAVALLAR, Guenter, 88326 Aulendorf (DE); GOEDERLE, Hubert, 88364 Wolfegg/Alttann (DE); STEIB, Janosch, 88444 Ummendorf (DE)
(74) Vertreter: Gleiss, Alf-Olav
(86) Internationale Anmeldenummer: PCT/EP2008/009268
(87) Internationale Veröffentlichungsnummer: WO 2009/059733

(56) Entgegenhaltungen:
- EP-A- 1 240 914
- WO-A-03/090822
- WO-A-2007/065339
- US-A- 5 137 516

## Beschreibung

Die Erfindung betrifft eine Vorrichtung zur Montage einer pharmazeutischen Applikationshilfe nach dem Oberbegriff des Anspruchs 1 sowie ein Verfahren zur Montage einer pharmazeutischen Applikationshilfe nach dem Oberbegriff des Anspruchs 11. Die Erfindung betrifft auch die Verwendung einer Vorrichtung zur Montage einer pharmazeutischen Applikationshilfe gemäß Anspruch 14, sowie die Verwendung einer Vorrichtung zur Durchführung eines Verfahrens gemäß Anspruch 15.

Applikationshilfen der hier angesprochenen Art und Vorrichtungen (WO 2007/065339 A1) sowie Verfahren (EP 1 240 914 A) zu deren Montage sind bekannt. Dabei handelt es sich insbesondere um sogenannte Pens oder Autoinjektoren, die typischerweise Anwendung in der Insulintherapie bei Diabetes Mellitus finden. Applikationshilfen wie Pens ermöglichen einen einfacheren Transport, eine unkomplizierte Handhabung durch einen Benutzer und eine genaue Dosierung. Ein Vorteil von Pens ist auch die unauffällige Anwendung. Erhältlich sind Einmal-Pens, die fertig befüllt sind und eine nicht austauschbare, als Karpule ausgebildete Ampulle enthalten. Auch sind Pens erhältlich, deren Ampulle austauschbar ist und die mehrere Dosen eines bestimmten Medikaments abgeben kann. Bei den eingesetzten Karpulen kann es sich um Karpulen für normale Spritzenanwendungen, aber auch um spezielle Doppelkammerkarpulen zur Aufnahme eines Lyophilisats handeln. Applikationshilfen der hier angesprochenen Art umfassen typischerweise eine erste Gehäusehülse, in welche die Karpule eingebracht wird und eine zweite Gehäusehülse, die mit der ersten Gehäusehülse beispielsweise über eine Schraub- oder eine Steckverbindung zusammengefügt wird. In der zweiten Gehäusehülse ist eine Kolbenstange angeordnet, die zur Verlagerung eines Stopfens der Karpule und damit zur Injektion dient. Bei der vollautomatischen Montage derartiger Applikationshilfen sind Vorrichtungen zum maschinellen Zusammenfügen der beiden Gehäusehülsen vorgesehen, die eine exakte Positionierung der beiden Gehäusehülsen zueinander ermöglichen. Bei der halbautomatischen Montage derartiger Applikationshilfen, wird die zweite Gehäusehülse dagegen von Hand auf die erste Gehäusehülse aufgeschraubt oder auch aufgesteckt. Die halbautomatische Montage von Applikationshilfen wird insbesondere dann eingesetzt, wenn nur geringe Stückzahlen hergestellt werden sollen, bei denen sich der Einsatz einer vollautomatischen Montageeinheit aufgrund der erheblichen Kosten nicht lohnt.

Es hat sich bei der halbautomatischen Montage von Applikationshilfen gezeigt, dass die zweite Gehäusehülse bei der manuellen Montage oft zu weit auf die erste Gehäusehülse aufgeschraubt oder aufgesteckt wird, sodass die in der zweiten Gehäusehülse angeordnete Kolbenstange den Stopfen der in der ersten Gehäusehülse angeordneten Karpule verlagert. Durch die Verlagerung des Stopfens kann es dann zu einer Aktivierung beispielsweise eines Lyophilisats in einer Doppelkammerkarpule oder zum Austritt eines Injektionspräparats aus einer herkömmlichen Karpule kommen. Eine derartige Aktivierung des Injektionspräparats vor der eigentlichen Benutzung durch den Patienten ist unerwünscht und führt dazu, dass die Applikationshilfe unbrauchbar wird. Insbesondere besteht dabei die Gefahr, dass eine Aktivierung des Karpuleninhalts vor allem bei Doppelkammerkarpulen unbemerkt bleibt.

Aufgabe der vorliegenden Erfindung ist es daher, eine Vorrichtung zur Montage einer pharmazeutischen Applikationshilfe, insbesondere eines Pens zu schaffen, die den oben genannten Nachteil vermeidet, also beispielsweise ein zu weites Ineinanderdrehen zweier Gehäusehülsen bei der Montage einer Applikationshilfe vermeidet.

Zur Lösung dieser Aufgabe wird eine Vorrichtung zur Montage einer pharmazeutischen Applikationshilfe, insbesondere eines Pens, mit den Merkmalen des Anspruchs 1 vorgeschlagen. Die Vorrichtung umfasst eine Haltevorrichtung zur Fixierung einer ersten Gehäusehülse und einen Anschlag zur Begrenzung einer Verlagerung einer zweiten Gehäusehülse relativ zu der ersten Gehäusehülse. Sie zeichnet sich durch wenigstens einen ersten Sensor aus, der eine bestimmte Position der ersten Gehäusehülse im Bereich der Haltevorrichtung erfasst, und der mit der Haltevorrichtung zusammenwirkt und diese aktiviert, wenn die erste Gehäusehülse eine erste bestimmte Position erreicht. Durch die vorteilhafte Kombination einer Haltevorrichtung zum axialen und radialen Fixieren der ersten Gehäusehülse mit einem Anschlag zur Begrenzung des Verlagerungswegs der zweiten Gehäusehülse und mit einem Sensor zur Aktivierung der Haltevorrichtung, wird eine genaue Positionierung der Gehäusehülsen und damit eine zu weite Verlagerung der zweiten Gehäusehülse relativ zu der ersten Gehäusehülse beim Zusammenfügen der beiden Hülsen verhindert. Die Haltevorrichtung fixiert dabei die erste Gehäusehülse in einer axialen Position. Bei einer Schraubverbindung zwischen den beiden Gehäusehülsen erfolgt vorzugsweise zusätzlich eine radiale Fixierung der ersten Hülse durch die Haltevorrichtung. Wird nun die zweite Gehäusehülse mit der ersten Gehäusehülse zusammengefügt, beispielsweise durch eine Schraub- oder Steckverbindung, kann die Verlagerung der zweiten Gehäusehülse nur bis zu dem Anschlag erfolgen. Eine zu weite Verlagerung, beispielsweise durch eine Umdrehung zu viel oder ein zu weites Aufstecken der zweiten Gehäusehülse wird somit effektiv durch den Anschlag verhindert. Dadurch, dass die erste Gehäusehülse in der Haltevorrichtung fixiert ist, kann sich diese auch nicht nach oben in die erste Gehäusehülse hineinverlagern, insbesondere dann nicht, wenn eine Schraubverbindung vorgesehen ist. Durch die sensoraktivierte Haltevorrichtung für die erste Gehäusehülse in Verbindung mit dem Anschlag für die zweite Gehäusehülse wird somit eine vorteilhafte Sicherung gegen die Aktivierung eines Karpuleninhalts geschaffen. Für die erste Gehäusehülse kann zusätzlich zu dem ersten Sensor ein Anschlagselement vorgesehen sein, das sich im Bereich des ersten Sensors befindet. Es ist also sehr wohl denkbar, ohne Anschlagselement ausschließlich durch die Aktivierung der Haltevorrichtung mittels des Sensors die erste Gehäusehülse in einer bestimmten Position zu halten. Um eine exakte Positionierung der ersten Gehäusehülse zu erreichen, ist ein Anschlagselement jedoch von Vorteil. Insgesamt ist die Verwendung einer Vorrichtung zur Montage einer pharmazeutischen Applikationshilfe, wobei die Vorrichtung die oben genannten Merkmale aufweist, besonders vorteilhaft.

Eine besonders bevorzugte Vorrichtung zeichnet sich dadurch aus, dass die Haltevorrichtung pneumatisch, hydraulisch oder magnetisch betrieben wird. Denkbar ist jedoch auch eine manuelle Betätigung. Die Ausgestaltung der Haltevorrichtung ist prinzipiell beliebig. Entscheidend ist, dass sie die erste Gehäusehülse sicher in einer bestimmten Position axial und radial fixiert.

Bei einer weiteren bevorzugten Vorrichtung ist vorgesehen, dass eine Aufnahmeeinheit zur Aufnahme der ersten Gehäusehülse vorgesehen ist. Vorzugsweise umschließt die Aufnahmeeinheit die erste Gehäusehülse zumindest bereichsweise. Die Aufnahmeeinheit dient insbesondere zur Führung der ersten Gehäusehülse, bis diese von dem ersten Sensor erfasst wird und/oder an dem Anschlagselement anstößt. Durch die Aufnahmeeinheit ist ferner eine exakte Ausrichtung der ersten Gehäusehülse hinsichtlich der Haltevorrichtung möglich.

Eine weitere bevorzugte Vorrichtung zeichnet sich dadurch aus, dass die Haltevorrichtung wenigstens bereichsweise in die Aufnahmeeinheit eingreift. Dabei können beispielsweise Öffnungen in der Aufnahmeeinheit vorgesehen sein, durch die Greifelemente der Haltevorrichtung greifen können, um die erste Gehäusehülse zu fixieren. Darüber hinaus sind noch zahlreiche weitere Ausgestaltungen der Haltevorrichtung und der Aufnahmeeinheit möglich, denkbar ist beispielsweise auch die einstückige Ausbildung der Haltevorrichtung mit der Aufnahmeeinheit.

Auch wird eine Vorrichtung bevorzugt, die sich dadurch auszeichnet, dass der Anschlag für die zweite Gehäusehülse einstückig mit der Aufnahmeeinheit ausgebildet ist. Insbesondere kann vorgesehen sein, dass an der Aufnahmeeinheit eine Schulter ausgebildet ist, die als Anschlag wirkt. Der Anschlag kann jedoch auch getrennt von der Aufnahmeeinheit angeordnet sein, darüber hinaus ist auch eine einstückige Ausbildung des Anschlags mit der Haltevorrichtung denkbar.

Eine weitere bevorzugte Vorrichtung zeichnet sich dadurch aus, dass wenigstens ein zweiter Sensor vorgesehen ist, der das Anliegen der zweiten Gehäusehülse an dem Anschlag erfasst. Wird also die zweite Gehäusehülse mit der ersten Gehäusehülse zusammengefügt, beispielsweise verschraubt, so kann vorgesehen sein, dass nach einer Umdrehung der zweiten Gehäusehülse diese an dem Anschlag anliegt. Der zweite Sensor, der sich vorzugsweise im Bereich des Anschlags befindet, erfasst dann die Position der zweiten Gehäusehülse an dem Anschlag. Insbesondere kann vorgesehen sein, dass der zweite Sensor mit der Haltevorrichtung zusammenwirkt und nach dem Erfassen der zweiten Gehäusehülse im Bereich des Anschlags die Haltevorrichtung deaktiviert.

Besonders bevorzugt wird auch eine Vorrichtung, bei der der Anschlag für die zweite Gehäusehülse und das Anschlagselement für die erste Gehäusehülse in einem definierten Abstand zueinander angeordnet sind. Der Abstand zwischen dem Anschlagselement und dem Anschlag ist entscheidend dafür, dass keine Verlagerung des Stopfens in der Karpule durch die in der zweiten Gehäusehülse angeordnete Kolbenstange erfolgt, wenn die zweite Gehäusehülse mit der ersten Gehäusehülse zusammengefügt wird. Je nach Länge der Gehäusehülsen und Ausmaße der Karpule kann der Abstand variieren.

Schließlich wird noch eine Vorrichtung bevorzugt, bei der wenigstens ein dritter Sensor vorgesehen ist, der die Position der Karpule in der ersten Gehäusehülse erfasst. Der dritte Sensor ist vorzugsweise als Laser-Sensor ausgebildet und erzeugt ein entsprechendes Signal, wenn die Karpule richtig in der Gehäusehülse angeordnet wurde.

Weitere Merkmale der Vorrichtung ergeben sich aus den Unteransprüchen.

Aufgabe der Erfindung ist es außerdem, ein Verfahren zur Montage einer pharmazeutischen Applikationshilfe, insbesondere eines Pens zu schaffen, welches den oben genannten Nachteil vermeidet, also beispielsweise ein zu weites Ineinanderdrehen zweier Gehäusehülsen bei der Montage einer Applikationshilfe vermeidet.

Zur Lösung dieser Aufgabe wird ein Verfahren zur Montage einer pharmazeutischen Applikationshilfe, vorzugsweise eines Pens vorgeschlagen, das die Merkmale des Anspruchs 11 aufweist. Es umfasst in einem ersten Schritt das Bereitstellen einer ersten Gehäusehülse, ferner das Aktivieren einer Haltevorrichtung zur Fixierung der ersten Gehäusehülse in einer bestimmten Position. Anschließend erfolgt das Einbringen eines pharmazeutischen Behältnisses, vorzugsweise einer Karpule, in die erste Gehäusehülse und das Zusammenfügen einer zweiten Gehäusehülse mit der ersten Gehäusehülse. In einem weiteren Schritt erfolgt das Verlagern der zweiten Gehäusehülse relativ zu der ersten Gehäusehülse bis zu einem mit der zweiten Gehäusehülse zusammenwirkenden Anschlag. Das Verfahren zeichnet sich dadurch aus, dass wenigstens ein erster Sensor das Erreichen der bestimmten Position der ersten Gehäusehülse erfasst, der mit der Haltevorrichtung zusammenwirkt und diese aktiviert, wenn die erste Gehäusehülse die bestimmte Position erreicht. Wenigstens ein zweiter Sensor erfasst das Anlegen der zweiten Gehäusehülse an dem Anschlag und wirkt mit der Haltevorrichtung zusammen, wobei er diese deaktiviert. Durch das hier vorgeschlagene Verfahren zur Montage einer Applikationshilfe wird in vorteilhafter Weise verhindert, dass beim Zusammenfügen der zweiten Gehäusehülse mit der ersten Gehäusehülse ein Überdrehen stattfindet, sodass beispielsweise der Inhalt einer Doppelkammerspritze aktiviert wird oder ein Injektionspräparat aus einer herkömmlichen Karpule austritt. Dadurch, dass die zweite Gehäusehülse nicht weiter als bis zu dem Anschlag relativ zu der ersten Gehäusehülse verlagert werden kann und die erste Gehäusehülse andererseits in einer bestimmten Position durch die Haltevorrichtung sowohl radial als auch axial fixiert ist, wird sichergestellt, dass eine Aktivierung des Karpuleninhalts, beispielsweise durch versehentliches zu weites Drehen der zweiten Gehäusehülse auf der ersten Gehäusehülse und eine damit verbundene Verlagerung des Karpulenstopfens vermieden wird. Erreicht die zweite Gehäusehülse den Anschlag, wird dies von dem zweiten Sensor erfasst, der im Übrigen mit der Haltevorrichtung zusammenwirken kann. Diese kann durch ein entsprechendes Signal des Sensors deaktiviert werden, sodass die fertig montierte Applikationshilfe aus der Vorrichtung entnommen werden kann. Besonders bevorzugt wird ein Verfahren, das sich dadurch auszeichnet, dass zum Erreichen der bestimmten Position die erste Gehäusehülse bis an ein Anschlagselement verlagert wird. Durch das Anschlagselement kann eine definierte Positionierung der ersten Gehäusehülse sowohl hinsichtlich des Anschlags als auch hinsichtlich der Haltevorrichtung erfolgen. Es kann weiterhin vorgesehen sein, dass ein erster Sensor das Erreichen der bestimmten Position erfasst. Dieser kann alternativ oder zusätzlich zu dem Anschlagselement vorgesehen sein. Denkbar ist es auch, die Positionierung der ersten Gehäusehülse ohne Anschlagselement vorzunehmen. Sobald die erste Gehäusehülse in den Wirkbereich des Sensors eintritt, muss dieser die Haltevorrichtung aktivieren, sodass eine weitere Verlagerung der zweiten Gehäusehülse nicht möglich ist.

Ein weiteres bevorzugtes Verfahren zeichnet sich dadurch aus, dass der erste und/oder der zweite Sensor mit mindestens einem Anzeigeelement zusammenwirken. Denkbar ist beispielsweise der Einsatz einer Kontrollleuchte, die das Erfassen beispielsweise des ersten Sensors der ersten Gehäusehülse und/oder das Erfassen des zweiten Sensors der zweiten Gehäusehülse dem Bedienpersonal anzeigt.

Weitere Merkmale des Verfahrens ergeben sich aus den Unteransprüchen.

Zur Lösung der oben genannten Aufgabe kann auch die erfindungsgemäße Vorrichtung dazu verwendet werden, um eine pharmazeutische Applikationshilfe zu montieren, wobei sich die oben beschriebenen Vorteile ohne Weiteres einstellen.

Zur Lösung der genannten Aufgaben kann auch eine Vorrichtung der oben genannten Art dazu verwendet werden, das beschriebene Verfahren zur Montage einer Applikationshilfe durchzuführen.

Die Erfindung wird im Folgenden anhand der Zeichnung näher erläutert. Es zeigen:
- Figur 1: eine Vorderansicht einer Vorrichtung zur Montage einer pharmazeutischen Applikationshilfe mit einer ersten Gehäusehülse;
- Figur 2: eine Vorderansicht einer Vorrichtung gemäß Figur 1 mit einer ersten Gehäusehülse und einer Karpule;
- Figur 3: eine perspektivische Darstellung einer Vorrichtung nach Anspruch 1 mit einer ersten Gehäusehülse und einer zweiten Gehäusehülse;
- Figur 4: eine Detaildarstellung der ersten Gehäusehülse und der zweiten Gehäusehülse in zusammengefügtem Zustand und
- Figur 5: eine Vorderansicht einer Vorrichtung nach Figur 1 mit einer fertig montierten Applikationshilfe.

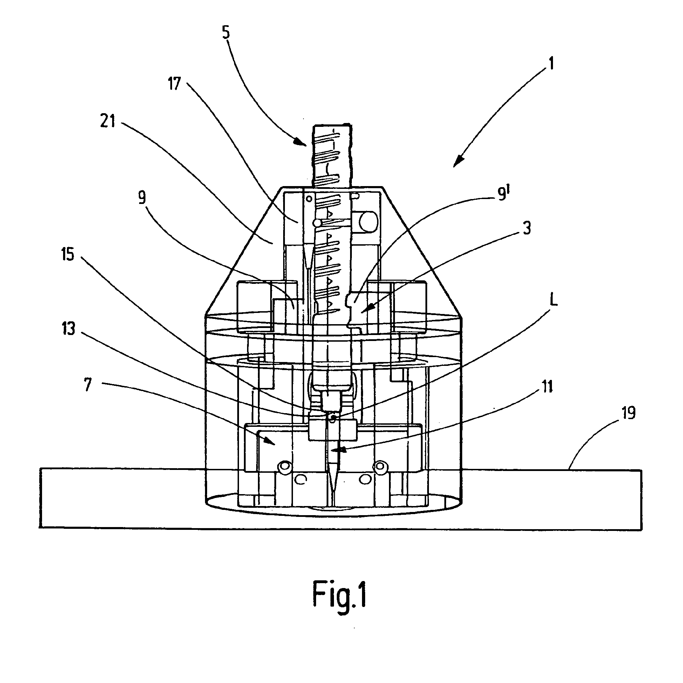

Figur 1 zeigt eine Vorrichtung 1 zur Montage einer pharmazeutischen Applikationshilfe. Bei der pharmazeutischen Applikationshilfe handelt es sich in dem in Figur 1 gezeigten Beispiel um einen sogenannten Autoinjektor oder Pen. Die Vorrichtung 1 umfasst eine Haltevorrichtung 3, die zur Fixierung einer ersten Gehäusehülse 5 in einer bestimmten axialen und/oder radialen Position dient.

Die Haltevorrichtung 3 kann auf verschiedene Art und Weise realisiert sein. Denkbar ist beispielsweise eine pneumatische, hydraulische oder magnetische aber auch eine manuelle Betätigung der Haltevorrichtung 3. In dem hier gezeigten Ausführungsbeispiel wird die Haltevorrichtung 3 durch einen Pneumatikzylinder 7 realisiert, der zwei Greifer 9 und 9' betätigt, welche die Fixierung der ersten Gehäusehülse 5 sowohl in radialer als auch in axialer Richtung bewirken. Der Angriffspunkt der Greifer 9 und 9' an der ersten Gehäusehülse 5 ist prinzipiell beliebig.

Die Greifer 9 und 9' werden bei der Aktivierung des wenigstens einen Pneumatikzylinders 7 aufeinander zu bewegt, sodass die erste Gehäusehülse 5 von den Greifern 9 und 9' fest umschlossen wird. Eine Fixierung der ersten Gehäusehülse 5 muss dabei derart erfolgen, dass eine axiale und eine radiale Verlagerung der ersten Gehäusehülse 5 verhindert werden. Eine Deaktivierung der Haltevorrichtung 3 führt zu einem Auseinanderbewegen der Greifer 9 und 9', sodass die erste Gehäusehülse 5 aus dem Bereich der Haltevorrichtung 3 entfernt werden kann.

Die Vorrichtung 1 weist wenigstens einen ersten Sensor 11 auf, der eine bestimmte Position der ersten Gehäusehülse 5 im Bereich der Haltevorrichtung 3 erfasst. Der erste Sensor 11 ist vorzugsweise als Laser-Sensor ausgebildet, der ein Signal erzeugt, wenn sein Laserstrahl unterbrochen wird. Er kann im Übrigen in vorteilhafter Weise dafür eingesetzt werden, die Aktivierung der Haltevorrichtung 3 auszulösen. Sobald die erste Gehäusehülse 5 so weit in die Vorrichtung 1 hineinverlagert ist, dass das untere Ende 13 der ersten Gehäusehülse 5 den Laserstrahl des Sensors 11 unterbricht, wird die Haltevorrichtung 3 aktiviert, sodass die erste Gehäusehülse 5 fixiert wird. In dem in Figur 1 gezeigten Beispiel verläuft ein Laserstrahl senkrecht zur Bildebene im Bereich des mit L gekennzeichneten Punkts.

Gleichzeitig oder auch alternativ zu dem ersten Sensor 11 kann ein Anschlagselement 15 vorgesehen sein, welches in Figur 1 nur angedeutet ist. Das Anschlagselement 15 dient dazu, den Verlagerungsweg der ersten Gehäusehülse 5 in der Vorrichtung 1 zu begrenzen und außerdem dazu, eine definierte Relativposition zwischen der ersten Gehäusehülse 5 und der Haltevorrichtung 3 zu schaffen. Der Laserstrahl im Punkt L ist vorzugsweise im Bereich des Anschlagselements 15 angeordnet, sodass also eine erste Gehäusehülse 5, die von Hand in die Vorrichtung 1 eingeführt wird und das Anschlagselement 15 erreicht, von dem ersten Sensor 11 erfasst wird und ein entsprechendes Signal zur Aktivierung der Haltevorrichtung 3 erzeugt.

Grundsätzlich denkbar ist auch eine Betätigung der Haltevorrichtung 3 durch das Bedienpersonal, das die erste Gehäusehülse 5 so weit in die Vorrichtung 1 hineinverlagert, bis diese an dem Anschlagselement 15 anliegt und somit eine weitere Verlagerung nicht mehr möglich ist. Dabei kann es allerdings zu einem Verkanten der ersten Gehäusehülse 5 kommen, beispielsweise in einer Aufnahmeeinheit 17, die insbesondere zur Führung der ersten Gehäusehülse 5 dient, sodass die Gehäusehülse 5 nicht oder zumindest nicht in der richtigen Position von der Haltevorrichtung 3 erfasst wird. Der erste Sensor 11 dient daher zur Absicherung, dass die erste Gehäusehülse 5 in der richtigen Position relativ zu der Haltevorrichtung 3 angeordnet ist. Andererseits ist es auch denkbar, ausschließlich einen ersten Sensor 11 zur Positionierung der ersten Gehäusehülse 5 vorzusehen, wie oben bereits erläutert wurde. Entscheidend ist, dass die erste Gehäusehülse 5 in einer definierten Position von der Haltevorrichtung 3 fixiert wird.

Die Aufnahmeeinheit 17 dient insbesondere zur Aufnahme der ersten Gehäusehülse 5 und zu deren Führung. Sie umschließt die erste Gehäusehülse 5 zumindest bereichsweise, wie in Figur 1 dargestellt ist. Die Haltevorrichtung 3 kann im Übrigen in die Aufnahmeeinheit 17 eingreifen, beispielsweise durch Öffnungen, die in der Aufnahmeeinheit 17 vorgesehen sind und durch welche die Greifer 9, 9' der Haltevorrichtung 3 an der ersten Gehäusehülse 5 angreifen können.

Die Vorrichtung 1 weist vorzugsweise noch einen Grundkörper 19 auf, in dem beispielsweise elektrische und/oder pneumatische oder sonstige Mittel angeordnet sind. Die in Figur 1 gezeigte Vorrichtung 1 weist außerdem eine Ummantelung 21 auf, die einerseits zum Schutz von sensorischen Elementen, wie dem ersten Sensor 11 und der Haltevorrichtung 3, dienen soll. Die Ummantelung 21 verbessert außerdem die optische Erscheinung der Vorrichtung 1 und stützt die Haltevorrichtung 3 sowie die Aufnahmeeinheit 17.

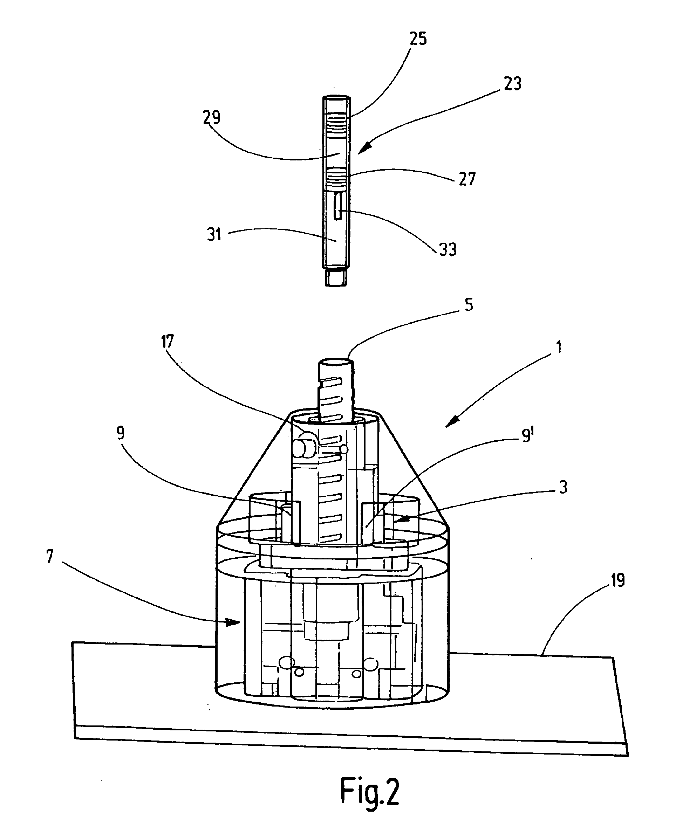

Figur 2 zeigt eine Vorderansicht der Vorrichtung 1 gemäß Figur 1. Gleiche Teile sind mit gleichen Bezugszeichen versehen, sodass insofern auf die Beschreibung zu Figur 1 verwiesen wird.

In Figur 2 ist wiederum die erste Gehäusehülse 5 in der Vorrichtung 1 angeordnet. Wie in der Figur angedeutet ist, wird in die von der Haltevorrichtung 3 gehaltene erste Gehäusehülse 5 eine Karpule 23 von Hand eingefügt. Es handelt sich hier beispielhaft um eine Doppelkammerkarpule; denkbar ist jedoch auch der Einsatz einer herkömmlichen Karpule. Die Doppelkammerkarpule umfasst einen ersten Stopfen 25 und einen zweiten Stopfen 27. Durch den zweiten Stopfen 27 werden zwei Kammern 29 und 31 voneinander getrennt, wobei sich in der Kammer 31 ein Lyophilisat und in Kammer 29 ein Fluid, vorzugsweise Wasser befindet.

Bei einer Verlagerung des ersten Stopfens 25 findet auch eine Verlagerung des Stopfens 27 statt, solange bis der Stopfen im Bereich eines Bypasses 33 angelangt ist. Über den Bypass 33 gelangt nun das Fluid aus Kammer 29 in die Kammer 31 um sich dort mit dem Lyophilisat zu vermischen. Eine Verlagerung des Stopfens 25 führt also zur Aktivierung der Injektionslösung der Karpule 23. Die Aktivierung der Karpule 23 soll erst dann erfolgen, wenn eine Injektion von einem Patienten vorgenommen wird.

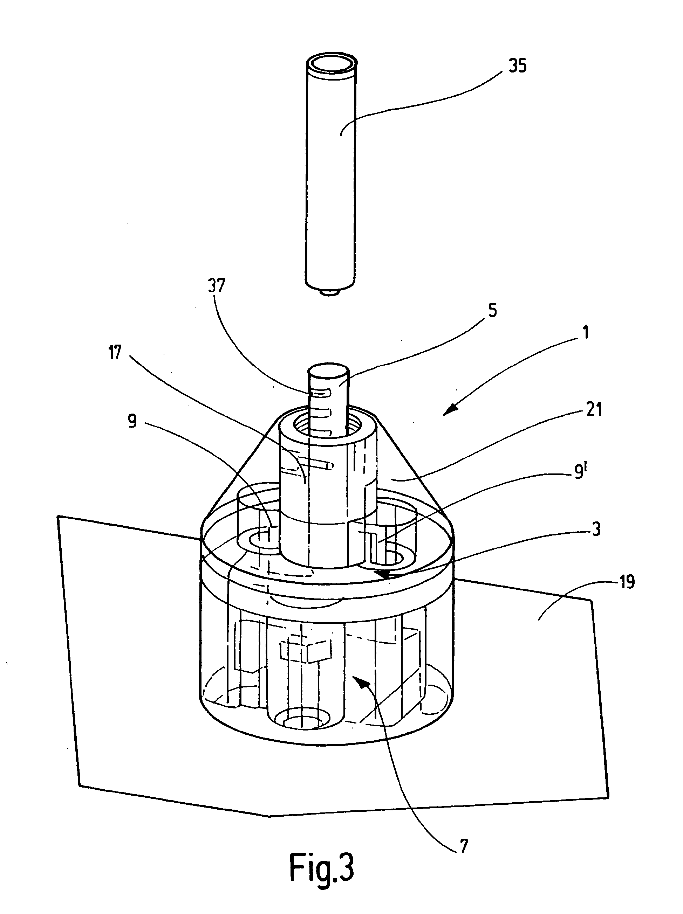

Figur 3 zeigt eine perspektivische Darstellung einer Vorrichtung 1 gemäß den Figuren 1 und 2. Gleiche Teile sind mit gleichen Bezugszeichen versehen, sodass insofern auf die Beschreibung zu den vorangegangenen Figuren verwiesen wird.

In der in Figur 3 dargestellten Vorrichtung 1 befindet sich die in Figur 2 gezeigte Karpule 23 in der ersten Gehäusehülse 5 und ist daher nicht mehr sichtbar. In Figur 3 dargestellt ist noch eine zweite Gehäusehülse 35, die zur fertigen Montage der Applikationshilfe mit der ersten Gehäusehülse 5 zusammengefügt werden muss. Rein beispielhaft werden die erste Gehäusehülse 5 und die zweite Gehäusehülse 35 hier mittels einer Schraubverbindung zusammengefügt. Die erste Gehäusehülse 5 umfasst zu diesem Zweck ein Außengewinde 37 und die zweite Gehäusehülse 35 ein hier nicht erkennbares entsprechendes Innengewinde. Ein Zusammenfügen der beiden Gewindehülsen 5 und 35 kann jedoch auch über eine Steckverbindung oder dergleichen erfolgen.

In der zweiten Gehäusehülse 35 ist eine nicht erkennbare Kolbenstange angeordnet, die dazu dient, im Bedarfsfall den in Figur 2 gezeigten Stopfen 25 zur Aktivierung der Karpule 23 zu verlagern. Bei der halbautomatischen Montage von Applikationshilfen, bei denen also die zweite Gehäusehülse 35 nicht maschinell, sondern manuell auf die erste Gehäusehülse 5 aufgesteckt oder aufgeschraubt wird, ist es von besonderer Bedeutung, dass die zweite Gehäusehülse 35 nicht zu weit auf die erste Gehäusehülse 5 aufgeschraubt/aufgesteckt wird, da ansonsten die in der zweiten Gehäusehülse 35 angeordnete Kolbenstange mit dem in Figur 2 gezeigten ersten Stopfen 25 in Kontakt kommt und diesen in der Karpule 23 verlagert, wodurch sich das Lyophilisat mit der Flüssigkeit vermischt. Bei einer nicht dargestellten herkömmlichen Karpule könnte es außerdem dazu kommen, dass während der Montage Injektionsflüssigkeit aus der Karpule austritt. Um dies zu verhindern, soll die zweite Gehäusehülse 35 nur mit einer bestimmten Anzahl von Umdrehungen, beispielsweise mit einer Umdrehung auf die erste Gehäusehülse 5 aufgeschraubt werden. Bei der manuellen Montage kann es hier jedoch sehr leicht zu einem Überdrehen kommen.

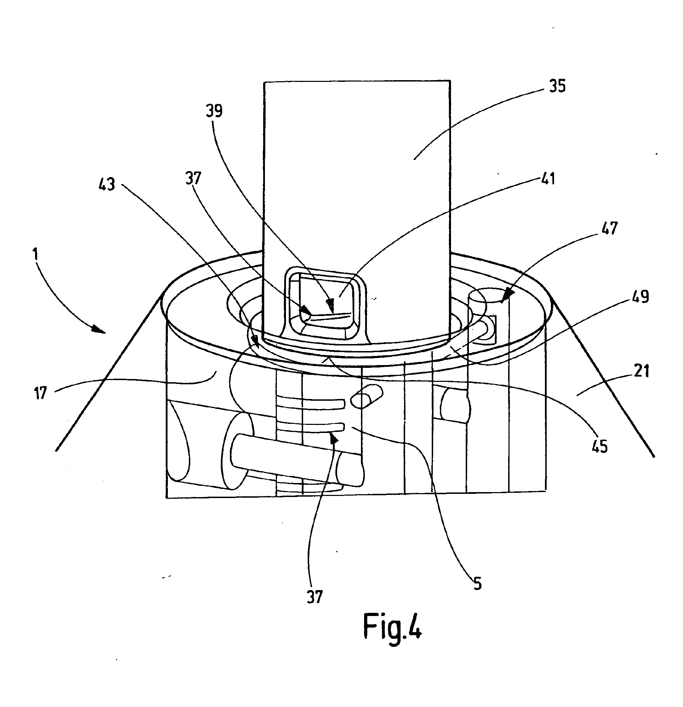

Figur 4 zeigt eine vergrößerte Darstellung des oberen Endabschnitts der zweiten Gehäusehülse 35, die mit der ersten Gehäusehülse 5 zusammengefügt wurde. Gleiche Teile sind mit gleichen Bezugszeichen versehen, sodass insofern auf die Beschreibung zu den vorangegangenen Figuren verwiesen wird.

Bei dem Aufschrauben der zweiten Gehäusehülse 35 auf die erste Gehäusehülse 5 erfolgt nach einer Umdrehung ein Einrasten eines Gewindeabschnitts 39 des Außengewindes 37 der ersten Gehäusehülse 5 in einer Öffnung 41 der zweiten Gehäusehülse 35. Um ein weiteres Verdrehen der zweiten Gehäusehülse 35 durch das Bedienpersonal zu verhindern, ist ein Anschlag 43 vorgesehen, an den das untere Ende 45 der zweiten Gehäusehülse 35 anstößt, sobald die zweite Gehäusehülse 35 mit einer Umdrehung an der ersten Gehäusehülse 5 befestigt wurde. Der Anschlag 43 kann auch erst nach zwei oder mehr Umdrehungen der zweiten Gehäusehülse 35 auf der ersten Gehäusehülse 5 vorgesehen sein. Entscheidend ist, dass ein Weiterdrehen der zweiten Gehäusehülse 35 durch den Anschlag 43 effektiv verhindert wird.

Der Abstand zwischen dem in Figur 4 nicht dargestellten Anschlagselement 15 und dem Anschlag 43 ist so dimensioniert, dass bei einem Anliegen des unteren Endes 45 der zweiten Gehäusehülse 35 an dem Anschlag 43 keine Verlagerung des ersten Stopfens 25 der Karpule 23 durch die nicht dargestellte in der zweiten Gehäusehülse 35 angeordnete Kolbenstange erfolgt.

Das Zusammenfügen der zweiten Gehäusehülse 35 mit der ersten Gehäusehülse 5 ist nicht auf eine Schraubverbindung beschränkt, es kann sich vielmehr auch um eine Steckverbindung mit Rastelementen handeln. Auch andere Verbindungsarten zwischen den beiden Gehäusehülsen sind denkbar. Entscheidend ist lediglich, dass eine relative Verlagerung zwischen der ersten Gehäusehülse 5 und der zweiten Gehäusehülse 35 durch den Anschlag 43 und die Haltevorrichtung 3 begrenzt wird.

In dem in Figur 4 dargestellten Beispiel ist der Anschlag 43 einstückig mit der Aufnahmeeinheit 17 ausgebildet. Der Anschlag 43 ist dort in Form einer Schulter in die Aufnahmeeinheit 17 eingebracht. Denkbar sind jedoch auch andere Realisierungen des Anschlags 43, beispielsweise durch ein freistehendes Element oder durch eine einstückige Ausbildung mit der Haltevorrichtung 3.

Gemäß Figur 4 weist die Vorrichtung 1 noch einen zweiten Sensor 47 auf, der vorzugsweise als Laser-Sensor ausgebildet und der im Bereich des Anschlags 43 angeordnet ist. Insbesondere ist vorgesehen, dass der zweite Sensor 47 ein Signal erzeugt, sobald dessen Laserstrahl 49 von der zweiten Gehäusehülse 35 unterbrochen wird, wenn diese mit der ersten Gehäusehülse 5 zusammengefügt wird. Der zweite Sensor 47 erfasst also das Anliegen der zweiten Gehäusehülse 45 an dem Anschlag 43. Der zweite Sensor 47 wirkt vorzugsweise mit der in Figur 4 nicht dargestellten Haltevorrichtung 3 zusammen und deaktiviert diese, wenn die zweite Gehäusehülse 35 den Anschlag 43 erreicht hat.

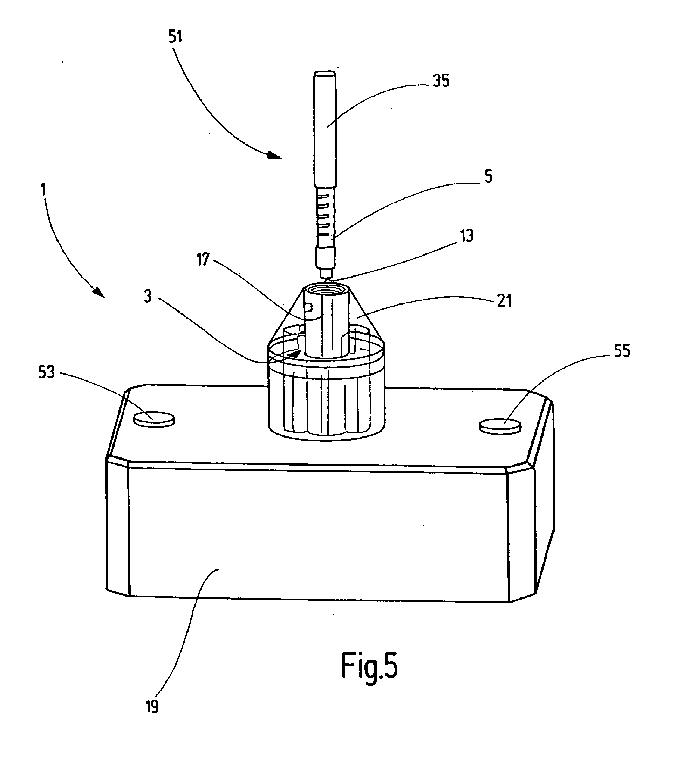

Figur 5 zeigt die Vorrichtung 1 gemäß den Figuren 1 bis 3. Gleiche Teile sind mit gleichen Bezugszeichen versehen, sodass insofern auch die Beschreibung zu den vorangegangenen Figuren verwiesen wird.

Figur 5 macht deutlich, dass nach dem Zusammenfügen der zweiten Gehäusehülse 35 mit der ersten Gehäusehülse 5 die Applikationshilfe 51 aus der Vorrichtung 1 entnommen werden kann. Bei Bedarf einer Injektion mittels des Pens kann der Benutzer die zweite Gehäusehülse 35 weiter auf die erste Gehäusehülse 5 schrauben, um eine Aktivierung des Karpuleninhalts auszulösen.

Es wird noch deutlich, dass die Haltevorrichtung 3, die Aufnahmeeinheit 17 und die Ummantelung 29 auf dem Grundkörper 19 angeordnet sind. Ferner sind an dem Grundkörper 19 Anzeigeelemente 53 und 55 vorgesehen, die vorzugsweise mit dem nicht erkennbaren ersten Sensor 11 und dem zweiten Sensor 47 zusammenwirken. Beispielsweise kann vorgesehen sein, dass, sobald der erste Sensor 11 die korrekte Position der ersten Gehäusehülse 5 in der Vorrichtung 1 erfasst hat, ein entsprechendes Signal an eine der Anzeigeelemente 53 oder 55 übermittelt wird, um dem Bedienpersonal anzuzeigen, dass die Haltevorrichtung 3 die erste Gehäusehülse 5 fixiert und ein Zusammenfügen mit der zweiten Gehäusehülse 35 nun möglich ist.

Auch ist es denkbar, dass der zweite Sensor 47 ein Signal an eines der Anzeigeelement 53 oder 55 übermittelt, um dem Bedienpersonal anzuzeigen, dass die zweite Gehäusehülse 35 bis zu dem Anschlag 43 auf die erste Gehäusehülse 5 aufgebracht wurde.

Denkbar ist auch die Realisierung von akustischen Signalen, die dem Bedienpersonal die entsprechenden Zustände der Vorrichtung 1 anzeigen. Nach dem Zusammenfügen der beiden Gehäusehülsen 5 und 35 ist es auch denkbar, dass ein entsprechendes Signal von dem zweiten Sensor 47 an die Haltevorrichtung 3 übermittelt wird, um diese zu deaktivieren. Aus Sicherheitsgründen ist jedoch auch die manuelle Deaktivierung der Haltevorrichtung 3 durch das Bedienpersonal denkbar.

Aus den Erläuterungen zu den Figuren wird deutlich, dass hier eine spezielle Applikationshilfen-Montagevorrichtung geschaffen wurde, die dazu dient, Applikationshilfen mit einer sehr hohen Fertigungssicherheit herzustellen. Insbesondere können Behälter, die der Aufnahme eines pharmazeutischen Produkts dienen, mit hoher Sicherheit in die Applikationshilfe eingebracht werden. Sie werden dazu in zwei Gehäusehülsen eingebracht, die gemeinsam einen Hohlraum zur Aufnahme des Behältnisses umschließen und so ausgebildet sind, dass das pharmazeutische Produkt aus dem Behälter ausgetragen werden kann. Dabei ist es auch möglich, einen als Zwei-Kammer-System ausgebildeten Behälter zunächst zu aktivieren und ein Lyophilisat mit einem Lösungsmittel zu vermischen, um ein pharmazeutisches Produkt herzustellen. Dies kann dann während eines oder mehrerer Applikationsschritte einem Patienten zugeführt werden.

Die Applikationshilfen-Montagevorrichtung gewährleistet, dass der von den Gehäusehülsen aufgenommene Behälter bei der Montage der Applikationshilfe nicht beeinträchtigt wird, sei es dadurch, dass bereits das pharmazeutische Produkt zumindest teilweise ausgetragen oder ein Zwei-Kammer-System schon ungewollt während der Montage der pharmazeutischen Applikationshilfe aktiviert wird.

Im Folgenden soll näher auf die Funktion der hier beschriebenen Vorrichtung und auf das Verfahren zur Montage einer pharmazeutischen Applikationshilfe, vorzugsweise eines Pens oder eines Autoinjektors eingegangen werden.

Das vorteilhafte Verfahren erfordert zunächst das Bereitstellen einer ersten Gehäusehülse 5. In dem in Figur 1 gezeigten Ausführungsbeispiel der Vorrichtung 1 erfolgt das Bereitstellen der ersten Gehäusehülse 5 durch die Aufnahmeeinheit 17 und das angedeutete Anschlagselement 15, sodass die erste Gehäusehülse 5 also in der Aufnahmeeinheit 17 geführt wird, bis es das Anschlagselement 15 erreicht.

Das Verfahren umfasst ferner das anschließende Aktivieren der Haltevorrichtung 3 zur Fixierung der ersten Gehäusehülse 5 in einer bestimmten Position. Zum Erreichen der bestimmten Position wird die Gehäusehülse 5 vorzugsweise bis an das Anschlagselement 15 verlagert. Zusätzlich oder auch alternativ zu dem Anschlagselement 15 kann vorgesehen sein, dass der erste Sensor 11 das Erreichen der bestimmten Position durch die erste Gehäusehülse 5 erfasst. Eine Aktivierung der Haltevorrichtung 3 kann entweder manuell durch den Bediener oder automatisch über ein entsprechendes Signal des Sensors 11 erfolgen.

Zur Fertigstellung der Applikationshilfe 51 ist ferner das Einbringen eines pharmazeutischen Behältnisses, vorzugsweise einer Karpule 23, in die erste Gehäusehülse 5 notwendig. Bei dem pharmazeutischen Behältnis kann es sich um ein auch als Doppelkammerkarpule bezeichnetes Zwei-Kammer-System oder um eine herkömmliche Karpule handeln.

Nach dem Fixieren der ersten Gehäusehülse 5 durch die Haltevorrichtung 3 wird diese mit der zweiten Gehäusehülse 35 zusammengefügt. Dies kann beispielsweise über eine Schraubverbindung oder über eine Steckverbindung erfolgen. Nach dem Zusammenfügen der beiden Gehäusehülsen 5 und 35 folgt die Verlagerung der zweiten Gehäusehülse 35 relativ zu der ersten Gehäusehülse 5, bis die zweite Gehäusehülse 35 den Anschlag 34 erreicht. Durch die Fixierung der ersten Gehäusehülse 5 mittels der Haltevorrichtung 3 und andererseits das Anliegen der zweiten Gehäusehülse 35 an dem Anschlag 43 ist eine weitere Verlagerung der beiden Gehäusehülsen 5 und 35 aufeinander zu ausgeschlossen:

Zusätzlich kann vorgesehen sein, dass ein zweiter Sensor 47 das Erreichen des Anschlags 43 durch die zweite Gewindehülse 35 erfasst. Sowohl der erste Sensor 11 als auch der zweite Sensor 47 kann mit der Haltevorrichtung 3 zusammenwirken, wobei der erste Sensor 11 die Haltevorrichtung 3 aktiviert und der zweite Sensor 47 die Haltevorrichtung 3 deaktiviert. Beide Sensoren 11 und 47 können ferner mit Anzeigeelementen 53 und 55 zusammenwirken. Die Sensoren 11, 47 sind vorzugsweise als Laser-Sensoren ausgebildet, denkbar ist jedoch auch jede andere Art Sensor, die geeignet ist, die Positionen der Gehäusehülsen 5 und 35 zu erfassen. Insbesondere kann vorgesehen sein, mehrere erste und zweite Sensoren vorzusehen. Die Haltevorrichtung 3 umfasst vorzugsweise pneumatische Elemente, jedoch können auch hydraulische oder magnetische Elemente vorgesehen sein. Die Haltevorrichtung 3 ist somit auf unterschiedlichste Art und Weise realisierbar.

Im Übrigen kann noch ein nicht dargestellter dritter Sensor vorgesehen sein, der vorzugsweise durch die Öffnung der ersten Gehäusehülse 5 im Bereich des unteren Endes 13 erfasst, ob sich die Karpule 23 in der ersten Gehäusehülse 5 befindet und im Fall der Anwesenheit der Karpule 23 ein entsprechendes Signal erzeugt. Auch der dritte Sensor kann mit einem Anzeigeelement 53, 55, beispielsweise einer grünen LED oder dergleichen, zusammenwirken, sodass dem Bedienpersonal angezeigt wird, wenn die Karpule 23 an der richtigen Position in der ersten Gehäusehülse 5 angeordnet ist und die zweite Gehäusehülse 35 mit der ersten Gehäusehülse 5 zusammengefügt werden kann. Sollte die Karpule 23 nicht richtig in der ersten Gehäusehülse 5 platziert worden sein, kann ebenfalls ein entsprechendes Signal erzeugt und an ein entsprechendes Anzeigeelement 53, 55, beispielsweise eine rote LED, übermittelt werden. Ferner kann er ebenso wie der erste Sensor 11 und der zweite Sensor 47 als Laser-Sensor ausgebildet sein, jedoch ist auch jede andere geeignete Art Sensor einsetzbar. Es kann auch vorgesehen sein, für die hier beschriebene Kontrolle anstelle des dritten Sensors mehrere Sensoren vorzusehen.

Insgesamt zeigt sich, dass bei der hier vorgeschlagenen vorteilhaften Vorrichtung 1 zur Montage einer pharmazeutischen Applikationshilfe 51 mehrere, vorzugsweise drei Sensoren eine korrekte Positionierung sowohl der ersten und zweiten Gehäusehülse, als auch der Karpule 23 erfassen. Im Fehlerfall, wenn also eines der genannten Elemente nicht richtig positioniert wurde, übermittelt der entsprechende Sensor ein (Fehler-)Signal an ein zugehöriges Anzeigeelement 53, 55, das dem Bediener die falsche Positionierung durch ein optisches oder auch ein akustisches Signal anzeigt.

Eine fehlerhafte Positionierung der ersten Gehäusehülse 5 in der Haltevorrichtung 3 kann beispielsweise dann vorliegen, wenn sie ihre bestimmte Position, die in der Beschreibung zu Figur 1 näher erläutert wurde, nicht erreicht, beispielsweise durch ein Verkanten in der Aufnahmeeinheit 17. Eine fehlerhafte Positionierung der zweiten Gehäusehülse 35 hingegen kann vorliegen, wenn sie ihre Endposition, nämlich den Anschlag 43 nicht erreicht. Auch ist es denkbar, dass die Karpule 23 beim Einführen in die erste Gehäusehülse 5 in dieser verkantet und dadurch ihre korrekte Position in der ersten Gehäusehülse 5 nicht einnehmen kann.

Liegt wenigstens einer der oben beschriebenen Fehlerfälle vor, ist vorzugsweise vorgesehen, dass eine entsprechend geschulte Bedienperson den fehlerhaften Pen ausschließlich mittels eines nicht dargestellten Schlüsselschalters aus der Vorrichtung 1 entfernen kann. Die Bedienung des Schalters kann also erst mittels eines Schlüssels erfolgen, auf den nur bestimmte Personen Zugriff haben. Der Schalter kann dann beispielsweise einen hier nicht näher beschriebenen Sperrmechanismus deaktivieren, der aktiviert wird, sobald einer der Sensoren ein Fehlersignal erzeugt. Dadurch, dass nur bestimmte Personen einen fehlerhaften Pen mittels des Schlüsselschalters aus der Vorrichtung 1 entfernen können, wird vermieden, dass fehlerhafte Pens mit fehlerfreien Pens versehentlich vermischt werden.

Entscheidend ist, dass eine Fixierung der ersten Gehäusehülse 5 in einer bestimmten Position erfolgt, und dass die anschließende Verlagerung der zweiten Gehäusehülse 35 relativ zu der ersten Gehäusehülse 5 von einem Anschlag begrenzt wird.

Aus den Erläuterungen sowohl zur Vorrichtung zur Montage einer pharmazeutischen Applikationshilfe als auch zu dem Verfahren zur Montage einer derartigen Applikationshilfe wird deutlich, dass die bei der Montage einer Applikationshilfe bestehenden Probleme mittels herkömmlicher Vorrichtungen dadurch beseitigt werden können, wenn eine Vorrichtung der hier beschriebenen Art dazu verwendet wird, eine Applikationshilfe zu montieren. Insbesondere wird sichergestellt, dass ein pharmazeutisches Behältnis in zwei Gehäusehülsen eingeschlossen werden kann, ohne dass es zu Beschädigungen oder Beeinträchtigungen des Behältnisses kommt. Es ist also sichergestellt, dass Pharmazeutika aus dem Behältnis nicht bereits während der Montage der pharmazeutischen Applikationshilfe ausgetragen werden, wodurch die Applikationshilfe unbrauchbar würde. Vermieden wird auch, dass ein als Zwei-Kammer-System ausgebildetes Behältnis während der Montage der pharmazeutischen Applikationshilfe bereits ungewollt aktiviert wird und damit grundsätzlich unbrauchbar ist.

Darüber hinaus wird deutlich, dass bei der Verwendung einer hier beschriebenen Vorrichtung das Verfahren zur Montage einer pharmazeutischen Applikationshilfe problemlos durchführbar ist und dabei eine hohe Produktionssicherheit gewährleistet werden kann.

## Patentansprüche

1. Vorrichtung (1) zur Montage einer pharmazeutischen Applikationshilfe (51), insbesondere eines Pens, mit
- einer Haltevorrichtung (3) zur Fixierung einer, ein pharmazeutisches Behältnis, insbesondere eine Karpule (23) aufnehmenden ersten Gehäusehülse (5) und mit
- einem Anschlag (43) zur Begrenzung einer Verlagerung einer zweiten Gehäusehülse (35) relativ zu der ersten Gehäusehülse (5),
**gekennzeichnet durch**
wenigstens einen ersten Sensor (11), der eine bestimmte Position der ersten Gehäusehülse (5) im Bereich der Haltevorrichtung (3) erfasst, und der mit der Haltevorrichtung (3) zusammenwirkt und diese aktiviert, wenn die erste Gehäusehülse (5) die bestimmte Position erreicht.

2. Vorrichtung nach Anspruch 1, **dadurch gekennzeichnet, dass** die Haltevorrichtung (3) pneumatisch, hydraulisch, magnetisch oder manuell betätigbar ist.

3. Vorrichtung nach Anspruch 1 oder 2, **dadurch gekennzeichnet, dass** der wenigstens eine erste Sensor (11) als Laserlicht-Sensor ausgebildet ist.

4. Vorrichtung nach einem der vorhergehenden Ansprüche, **dadurch gekennzeichnet, dass** für die erste Gehäusehülse (5) ein Anschlagselement (15) vorgesehen ist.

5. Vorrichtung nach einem der vorhergehenden Ansprüche, **dadurch gekennzeichnet, dass** eine Aufnahmeeinheit (17) zur Aufnahme der ersten Gehäusehülse (5) vorgesehen ist, welche vorzugsweise die erste Gehäusehülse (5) zumindest bereichsweise umschließt.

6. Vorrichtung nach einem der vorhergehenden Ansprüche, **dadurch gekennzeichnet, dass** wenigstens ein vorzugsweise als Laserlicht-Sensor ausgebildeter zweiter Sensor (47) vorgesehen ist, der das Anliegen der zweiten Gehäusehülse (35) an dem Anschlag (43) erfasst, und der vorzugsweise mit der Haltevorrichtung (3) zusammenwirkt und diese deaktiviert.

7. Vorrichtung nach einem der vorhergehenden Ansprüche 4 bis 6, **dadurch gekennzeichnet, dass** der Anschlag (43) und das Anschlagselement (15) in einem definierten Abstand zueinander angeordnet sind.

8. Vorrichtung nach einem der vorhergehenden Ansprüche 5 bis 7, **dadurch gekennzeichnet, dass** ein Grundkörper (19) vorgesehen ist, auf dem die Haltevorrichtung (3) und die Aufnahmeeinheit (17) angeordnet sind, und der vorzugsweise der Aufnahme von elektrischen und/oder pneumatischen Elementen dient.

9. Vorrichtung nach einem der vorhergehenden Ansprüche, **dadurch gekennzeichnet, dass** wenigstens ein vorzugsweise als Laserlicht-Sensor ausgebildeter dritter Sensor vorgesehen ist, der die Position der Karpule (23) in der ersten Gehäusehülse (5) erfasst.

10. Vorrichtung nach einem der vorhergehenden Ansprüche, **dadurch gekennzeichnet, dass** wenigstens ein Anzeigeelement (53, 55) vorgesehen ist, welches mit dem ersten und/oder dem zweiten und/oder dem dritten Sensor (11, 47) zusammenwirkt.

11. Verfahren zur Montage einer pharmazeutischen Applikationshilfe (51), vorzugsweise eines Pens, insbesondere mittels einer Vorrichtung (1) nach einem der Ansprüche 1 bis 10, mit folgenden Schritten:
- Bereitstellen einer ersten Gehäusehülse (5);
- Aktivieren einer Haltevorrichtung (3) zur Fixierung der ersten Gehäusehülse (5) in einer bestimmten Position;
- Einbringen eines pharmazeutischen Behältnisses, vorzugsweise einer Karpule (23), in die erste Gehäusehülse (5);
- Zusammenfügen einer zweiten Gehäusehülse (35) mit der ersten Gehäusehülse (5);
- Verlagern der zweiten Gehäusehülse (35) relativ zu der ersten Gehäusehülse (5) bis zu einem mit der zweiten Gehäusehülse (35) zusammenwirkenden Anschlag (43),
**dadurch gekennzeichnet,**
- **dass** wenigstens ein erster Sensor (11) das Erreichen der bestimmten Position der ersten Gehäusehülse (5) erfasst, der mit der Haltevorrichtung (3) zusammenwirkt und diese aktiviert, wenn die erste Gehäusehülse (5) die bestimmte Position erreicht; und
- **dass** wenigstens ein zweiter Sensor (47) das Anliegen der zweiten Gehäusehülse (35) an dem Anschlag (43) erfasst und mit der Haltevorrichtung (3) zusammenwirkt und diese deaktiviert.

12. Verfahren nach Anspruch 11, **dadurch gekennzeichnet, dass** der wenigstens eine erste Sensor (11) als Laserlicht-Sensor ausgebildet ist, und/oder dass wenigstens ein vorzugsweise als Laserlicht-Sensor ausgebildeter zweiter Sensor (47) das Erreichen des Anschlags (43) erfasst und/oder wenigstens ein vorzugsweise als Laserlicht-Sensor ausgebildeter dritter Sensor vorgesehen ist, der die Position der Karpule (23) in der ersten Gehäusehülse (5) erfasst.

13. Verfahren nach Anspruch 11 oder 12, **dadurch gekennzeichnet, dass** der erste und/oder der zweite und/oder der dritte Sensor (11, 47) mit der Haltevorrichtung (3) zusammenwirkt/zusammenwirken, wobei der erste Sensor (11) die Haltevorrichtung (3) aktiviert und der zweite Sensor (47) die Haltevorrichtung (3) deaktiviert.

14. Verwendung einer Vorrichtung nach einem der Ansprüche 1 bis 10 zur Montage einer pharmazeutischen Applikationshilfe.

15. Verwendung einer Vorrichtung nach einem der Ansprüche 1 bis 10 zur Durchführung eines Verfahrens nach einem der Ansprüche 11 bis 13.

## Claims

1. A device (1) for mounting a pharmaceutical applicator (51), more particularly a pen, the device (1) having
- a holding device (3) for locating a first housing sleeve (5) that receives a pharmaceutical container, more particularly a carpule (23), and having
- a stop (43) for limiting a displacement of a second housing sleeve (35) in relation to the first housing sleeve (5),
**characterized by**
at least one first sensor (11) which registers a specific position of the first housing sleeve (5) in the vicinity of the holding device (3) and which cooperates with the holding device (3) and activates the latter when the first housing sleeve (5) arrives at the specific position.

2. The device according to claim 1, **characterized in that** the holding device (3) can be actuated pneumatically, hydraulically, magnetically, or manually.

3. The device according to claim 1 or 2, **characterized in that** the at least one first sensor (11) is designed as a laser light sensor.

4. The device according to any one of the preceding claims, **characterized in that** a stop element (15) is provided for the first housing sleeve (5).

5. The device according to any one of the preceding claims, **characterized in that** a receiving unit (17) is provided for receiving the first housing sleeve (5), said receiving unit (17) preferably enclosing the first housing sleeve (5) at least sectionally.

6. The device according to any one of the preceding claims, **characterized in that** at least one second sensor (47) that is preferably designed as a laser light sensor is provided, said second sensor (47) registering the abutment of the second housing sleeve (35) on the stop (43) and preferably cooperating with the holding device (3) and deactivating the latter.

7. The device according to any one of the preceding claims 4 to 6, **characterized in that** the stop (43) and the stop element (15) are arranged spaced apart from each other by a defined distance.

8. The device according to any one of the preceding claims 5 to 7, **characterized in that** a base body (19) is provided, with the holding device (3) and the receiving unit (17) being arranged on said base body (19) and said base body (19) preferably serving to receive electrical and/or pneumatical elements.

9. The device according to any one of the preceding claims, **characterized in that** at least one third sensor that is preferably designed as a laser light sensor is provided, said third sensor registering the position of the carpule (23) in the first housing sleeve (5).

10. The device according to any one of the preceding claims, **characterized in that** at least one indicator element (53, 55) is provided, said indicator element (53, 55) cooperating with the first and/or the second and/or the third sensor (11, 47).

11. A method for mounting a pharmaceutical applicator (51), preferably a pen, particularly by means of a device (1) according to any one of claims 1 to 10, the method comprising the steps of
- providing a first housing sleeve (5);
- activating a holding device (3) for locating the first housing sleeve (5) in a specific position;
- introducing a pharmaceutical container, preferably a carpule (23), into the first housing sleeve (5);
- joining of a second housing sleeve (35) with the first housing sleeve (5);
- displacing the second housing sleeve (35) in relation to the first housing sleeve (5) up to a stop (43) cooperating with the second housing sleeve (35),
**characterized in that**
- at least one first sensor (11) registers the arrival of the first housing sleeve (5) at the specific position, said first sensor (11) cooperating with the holding device (3) and activating the latter when the first housing sleeve (5) arrives at the specific position; and
- at least one second sensor (47) registers the abutment of the second housing sleeve (35) on the stop (43) and cooperates with the holding device (3) and deactivates the latter.

12. The method according to claim 11, **characterized in that** the at least one first sensor (11) is designed as a laser light sensor and/or that at least one second sensor (47) that is preferably designed as a laser light sensor registers the arrival at the stop (43) and/or at least one third sensor that is preferably designed as a laser light sensor is provided, said third sensor registering the position of the carpule (23) in the first housing sleeve (5).

13. The method according to claim 11 or 12, **characterized in that** the first and/or the second and/or the third sensor (11, 47) cooperate/cooperates with the holding device (3), wherein the first sensor (11) activates the holding device (3) and the second sensor (47) deactivates the holding device (3).

14. A use of a device according to any one of claims 1 to 10 for mounting a pharmaceutical applicator.

15. A use of a device according to any one of claims 1 to 10 for applying a method according to any one of claims 11 to 13.

## Revendications

1. Dispositif (1) pour le montage d'un applicateur pharmaceutique (51), notamment d'un stylo, comprenant
- un dispositif de maintien (3) pour la fixation d'un premier manchon de logement (5) recevant un récipient pharmaceutique, notamment une cartouche (23) et comprenant
- une butée (43) pour la limitation d'un déplacement d'un second manchon de logement (35) par rapport au premier manchon de logement (5),
**caractérisé par**
au moins un premier capteur (11) qui détecte une position déterminée du premier manchon de logement (5) dans la région du dispositif de maintien (3) et qui coopère avec le dispositif de maintien (3) et active celui-ci lorsque le premier manchon de logement (5) atteint la position déterminée.

2. Dispositif selon la revendication 1, **caractérisé en ce que** le dispositif de maintien (3) peut être actionné de manière pneumatique, hydraulique, magnétique ou manuelle.

3. Dispositif selon la revendication 1 ou 2, **caractérisé en ce que** l'au moins un premier capteur (11) est réalisé en tant que capteur à lumière laser.

4. Dispositif selon l'une quelconque des revendications précédentes, **caractérisé en ce qu'**un élément de butée (15) est prévu pour le premier manchon de logement (5).

5. Dispositif selon l'une quelconque des revendications précédentes, **caractérisé en ce qu'**un module de réception (17) pour la réception du premier manchon de logement (5), qui entoure de préférence au moins par région le premier manchon de logement (5), est prévu.

6. Dispositif selon l'une quelconque des revendications précédentes, **caractérisé en ce qu'**au moins un deuxième capteur (47) réalisé de préférence en tant que capteur à lumière laser, qui détecte l'appui du second manchon de logement (35) sur la butée (43) et qui coopère de préférence avec le dispositif de maintien (3) et désactive celui-ci, est prévu.

7. Dispositif selon l'une quelconque des revendications précédentes 4 à 6, **caractérisé en ce que** la butée (43) et l'élément de butée (15) sont disposés à un écart défini l'un par rapport à l'autre.

8. Dispositif selon l'une quelconque des revendications précédentes 5 à 7, **caractérisé en ce qu'**un corps de base (19), sur lequel le dispositif de maintien (3) et le module de réception (17) sont disposés et qui sert de préférence à la réception d'éléments électriques et/ou pneumatiques, est prévu.

9. Dispositif selon l'une quelconque des revendications précédentes, **caractérisé en ce qu'**au moins un troisième capteur réalisé de préférence en tant que capteur à lumière laser, qui détecte la position de la cartouche (23) dans le premier manchon de logement (5), est prévu.

10. Dispositif selon l'une quelconque des revendications précédentes, **caractérisé en ce qu'**au moins un élément d'affichage (53, 55), qui coopère avec le premier et/ou le deuxième et/ou le troisième capteur (11, 47), est prévu.

11. Procédé de montage d'un applicateur pharmaceutique (51), de préférence d'un stylo, notamment au moyen d'un dispositif (1) selon l'une quelconque des revendications 1 à 10, avec les étapes suivantes :
- mise à disposition d'un premier manchon de logement (5) ;
- activation d'un dispositif de maintien (3) pour la fixation du premier manchon de logement (5) dans une position déterminée ;
- introduction d'un récipient pharmaceutique, de préférence d'une cartouche (23), dans le premier manchon de logement (5) ;
- assemblage d'un second manchon de logement (35) avec le premier manchon de logement (5) ;
- déplacement du second manchon de logement (35) par rapport au premier manchon de logement (5) jusqu'à une butée (43) coopérant avec le second manchon de logement (35),
**caractérisé en ce**
- **qu'**au moins un premier capteur (11) détecte l'atteinte de la position déterminée du premier manchon de logement (5), qui coopère avec le dispositif de maintien (3) et active celui-ci lorsque le premier manchon de logement (5) atteint la position déterminée ; et
- **qu'**au moins un deuxième capteur (47) détecte l'appui du second manchon de logement (35) sur la butée (43) et coopère avec le dispositif de maintien (3) et désactive celui-ci.

12. Procédé selon la revendication 11, **caractérisé en ce que** l'au moins un premier capteur (11) est réalisé en tant que capteur à lumière laser et/ou qu'au moins un deuxième capteur (47) réalisé de préférence en tant que capteur à lumière laser détecte l'atteinte de la butée (43) et/ou au moins un troisième capteur réalisé de préférence en tant que capteur à lumière laser, qui détecte la position de la cartouche (23) dans le premier manchon de logement (5), est prévu.

13. Procédé selon la revendication 11 ou 12, **caractérisé en ce que** le premier et/ou le deuxième et/ou le troisième capteur (11, 47) coopère(nt) avec le dispositif de maintien (3), dans lequel le premier capteur (11) active le dispositif de maintien (3) et le deuxième capteur (47) désactive le dispositif de maintien (3).

14. Utilisation d'un dispositif selon l'une quelconque des revendications 1 à 10 pour le montage d'un applicateur pharmaceutique.

15. Utilisation d'un dispositif selon l'une quelconque des revendications 1 à 10 pour la réalisation d'un procédé selon l'une quelconque des revendications 11 à 13.
